# FASCICULE DE BREVET EUROPEEN

(11) **EP 2 288 619 B1**
(45) Date de publication et mention de la délivrance du brevet: **04.04.2012**
(21) Numéro de dépôt: 09766020.3
(22) Date de dépôt: 18.06.2009
(51) Int. Cl.: C07K 14/44, A61K 39/008

(54) **PEPTIDE DE SYNTHESE NON NATIF CYCLISE ET COMPLEXE DE PEPTIDES COMPRENANT LEDIT PEPTIDE CYCLISE, DESTINE A INDUIRE ET A CARACTERISER LA PREVENTION OU LE TRAITEMENT D'AFFECTIONS CHEZ LES MAMMIFERES**
ZYKLIERTES, NICHTNATIVES SYNTHETISCHES PEPTID UND PEPTIDKOMPLEX MIT BESAGTEM ZYKLIERTEM PEPTID ZUR VERANLASSUNG UND CHARAKTERISIERUNG DER VORBEUGUNG ODER BEHANDLUNG VON STÖRUNGEN BEI SÄUGETIEREN
CYCLIZED NON-NATIVE SYNTHETIC PEPTIDE AND COMPLEX OF PEPTIDES COMPRISING SAID CYCLIZED PEPTIDE, INTENDED TO INDUCE AND TO CHARACTERIZE THE PREVENTION OR TREATMENT OF CONDITIONS IN MAMMALS

(30) Priorité: 19.06.2008 FR 0803436
(43) Date de publication de la demande: 02.03.2011
(73) Titulaire: PAPIEROK, Gérard, 83400 HYERES LES PALMIERS (FR); Vincens, Serge, 13180 Gignac la Nerthe (FR)
(72) Inventeur: VICENS, Serge, F-13180 Gignac La Nerthe (FR); HOTTIN, Gregory, F-83250 La Londe Les Maures (FR)
(74) Mandataire: Marek, Pierre
(86) Numéro de dépôt international: PCT/FR2009/000730
(87) Numéro de publication internationale: WO 2009/153458

(56) Documents cités:
- WO-A-03/025012
- WO-A-2005/051989
- WO-A-2006/058243

### SEQUENCE LISTING

<110> ORIDAN INC.
<120> Peptide de synthèse non natif cyclisé et complexe de peptides comprenant ledit peptide cyclisé
<130> B118 12PCT 06
<150> FR 08/03436
   <151> 2008-06-19
<160> 3
<170> PatentIn version 3.3
<210> 1
   <211> 34
   <212> PRT
   <213> Leishmania sp.
<400> 1
   Glu Asp Glu His Lys Gly Lys Tyr Cys Arg Leu Gly Asn Asp Cys Arg
   1 5 10 15
   Thr Thr Glu Pro Thr Thr Thr Ala Thr Pro Arg Gly Thr Pro Thr Pro
   20 25 30
   Ala Pro
<210> 2
   <211> 16
   <212> PRT
   <213> Leishmania sp.
<400> 2
   Ala Ala Arg Cys Ala Arg Cys Arg Glu Gly Tyr Ser Leu Thr Asp Glu
   1 5 10 15
<210> 3
   <211> 16
   <212> PRT
   <213> leishmania sp.
<400> 3
   Ala Ala Ser Ser Thr Pro Ser Pro Gly Ser Gly Cys Glu Val Asp Gly
   1 5 10 15

## Description

La présente invention concerne un peptide de synthèse non natif cyclisé par pont disulfure et son mélange à deux autres peptides de synthèse non natifs ainsi qu'à un adjuvant, pour former un complexe destiné à induire et à caractériser la prévention ou le traitement d'affections chez les mammifères dont l'immunité protectrice dépend de la stimulation des lymphocytes de type Th1 et notamment d'un état d'hyperstimulation de type retardé.

Ce complexe est plus particulièrement remarquable en ce qu'il permet d'induire une production importante d'anticorps spécifiques d'isotype IgG2, permettant en cas d'affections liées à un état d'hyperstimulation de type retardé, de distinguer un animal ou humain infecté ou malade, d'un animal ou humain non infecté ou non malade mais vacciné ou traité.

Enfin la présente invention peut être utilisée comme réactif induisant une stimulation des lymphocytes de type Th1 notamment pour la prévention ou le traitement d'affections liées à un état d'hyperstimulation immédiate et comme réactifs de diagnostic in vitro permettant de détecter les anticorps marqueurs de vaccination ou de traitement, c'est-à-dire les IgG2 spécifiques du peptide cyclisé. Enfin, la présente invention concerne également le rôle neutralisant de ces IgG2 caractérisé par l'inhibition de la prolifération des formes promastigotes ou amastigotes de Leishmania.

De nombreuses situations pathologiques sont associées à un état immunitaire de type Th1 ou Th2.

Pour exemple, l'exacerbation de la voie Th2 correspondant à un état d'hyperstimulation immédiate induit certaines affections comme les dermites atopiques canines, les allergies et l'asthme.
L'obtention d'une guérison pour ces affections est effectuée par des immunostimulants permettant le passage d'un état de Type Th2 à un état immunitaire de type Th1.

D'autre part, cet état immunitaire Th1 est en pleine corrélation avec un état de résistance aux pathogènes intracellulaires, tels *Leishmania, Trypanosoma, Candida, Mycobacterium* et *Listeria.*

Concernant l'immunopathologie de l'asthme, la littérature au cours de la dernière décennie a désigné le lymphocyte T spécifique de l'allergène comme le chef d'orchestre de la réaction immuno allergique (Magnan. A et al. "Cytoknies, from atopy to athsma : the Th2 dogma revisited" Cell Mol Biol, 2001, 47, 679-687). Parmi les lymphocytes T, la sous population Th2 productrice d'IL4 semble au premier plan et nécessaire à l'induction de cette réaction. Il est dès lors naturel que des stratégies soient établies pour cibler spécifiquement les lymphocytes et en particulier les Th2.

Ce type de stratégie peut être également suivie pour les leishmanioses.

Les leishmanioses constituent un ensemble complexe d'infections parasitaires qui sévissent dans de vastes régions du globe (88 pays, répartis sur 4 des 5 continents, sont touchés) et qui sont responsables d'un large spectre de manifestations cliniques (cutanée, muco-cutanée et viscérale, celle-ci étant la forme la plus grave car mortelle en absence de traitement).

350 millions de personnes sont exposés au risque de les contracter; environ 12 millions seraient porteurs du parasite et l'incidence annuelle est estimée entre 2 et 2,5 millions de nouveaux cas, parmi lesquels 500 000 personnes atteintes de leishmaniose viscérale.

La leishmaniose viscérale (LV) est causée par les parasites du complexe *Leishmania donovani* (*L. infantum*/*chagasi* et *L. donovani*) qui, sont des parasites intracellulaires obligatoires du macrophage. Contrairement aux autres espèces de *Leishmania* causant les formes cutanées de la maladie (*L*. *major, L. brasiliensis...*), les leishmanies du complexe *donovani* présentent une capacité à disséminer à l'ensemble des organes profonds où elles se multiplient. Les patients atteints de LV présentent alors un tableau clinique associant une fièvre modérée mais persistante, une hépatosplenomégalie et une pancytopénie conduisant généralement au décès si aucun traitement spécifique n'est initié suffisamment rapidement.

Les canidés sauvages et les chiens sont les principaux réservoirs de *L*. *chagasi* en Amériques centrale et du sud, et de *L. infantum* dans le bassin méditerranéen, étendu à certaines régions du Moyen Orient et de l'Asie. Dans ces régions forcement endémiques pour la leishmaniose canine, l'incidence de la maladie chez l'homme est très faible.

Même si la LV est un problème important en Europe du Sud avec l'extension de la pandémie du SIDA et l'apparition d'un nombre croissant de co-infections *Leishmania*/HIV*,* elle est loin d'être contrôlée et devient très préoccupante en terme de problème de santé publique. Dans d'autres parties du monde notamment dans les zones d'endémie à *L. donovani:* elle est estimée a environ 1/5 de la population au Soudan et en Inde où des épidémies meurtrières ont fait des centaines de milliers de victimes ces dernières décennies. De plus la situation devient critique en Inde avec l'émergence de souches résistantes au antimoniés pentavalents (médicaments de première intention).

Le contrôle des leishmanioses passe nécessairement par la chimiothérapie. Cette dernière est malheureusement mise en péril par des traitements longs, toxiques et onéreux s'accompagnant de nombreux cas de rechute et par l'émergence des phénomènes de chimiorésistance. Il paraît aujourd'hui évident que le contrôle de cette endémie parasitaire dépendra de la découverte de nouveaux moyens de prévention et de traitements abordables pour les populations concernées, la vaccination étant le moyen le plus adapté.

Le fait dé pouvoir induire une immunité acquise à la réinfection chez un individu qui avait été volontairement infecté avec des parasites vivants a très vite montré que la vaccination contre ces parasitoses était possible, du moins pour les formes cutanées de leishmaniose. Ces pratiques, encore appelées leishmanisation, longtemps utilisées aux Moyen-Orient, en Iran et en Ex URSS, ont été par la suite abandonnées de part leur dangerosité.

Parmi les candidats vaccinaux proposés durant ces dernières décennies, on distingue chronologiquement les vaccins de première génération qui utilisent les promastigotes vivants, morts ou atténués qui chez la souris ont donné des résultats plus que mitigés. Puis, plus récemment, l'avènement des techniques de biologie moléculaire a permis le développement de vaccins à sous-unités ou constitués de protéines recombinantes, comme la GP63, la GP46, le Lipophophoglycanne (LPG), ..., principalement des recombinants de protéines majeures exposées à la surface du parasites. Certaines de ces protéines recombinantes ont conféré un bon niveau de protection à l'encontre d'une infection expérimentale chez la souris. Enfin, il existe les vaccins de troisième génération qui font appel à la génomique fonctionnelle et utilisent des transgènes comme des microorganismes transfectés avec le gène de la GP63 et des leishmanies recombinantes à virulence atténuée ou des vaccins à ADN.

A tous ces travaux trois constats :
- la vaccination contre les leishmanioses cutanées a fait l'objet de nombreuses études comparativement à celles concernant la leishmaniose viscérale. Toutefois, les modèles d'infection expérimentale pour la leishmaniose viscérale sont plus difficiles à mettre en oeuvre (notamment à cause de la difficulté d'infecter expérimentalement des rongeurs).
- des résultats parfois extrêmement intéressants ont été obtenus chez la souris. Mais il est difficile et hasardeux de transposer directement des résultats obtenus chez la souris à des modèles d'infection naturelle de la leishmaniose (chien, homme). Les travaux de Dunan *et al.* menés en 1989 en sont un exemple très démonstratif. Ces auteurs avaient obtenu une très bonne protection chez la souris à l'encontre d'une infection d'épreuve avec *L. infantum* en utilisant une fraction antigénique appelée LiF2. Cette même fraction, injectée chez des chiens, non seulement ne les protégeait pas mais plus grave augmentait leur susceptibilité à l'infection
- peu de candidats vaccin ont dépassé le stade expérimental (souris, hamsters) de part la difficulté à mettre en oeuvre des essais cliniques longs, coûteux et fastidieux.

Ainsi donc , quand il ne s'agit pas d'études sur la leishmaniose, cutanée mais sur la leishmaniose viscérale, les principaux travaux relatifs à la vaccination ont été et sont encore réalisés chez la souris. Toutefois, ces modèles d'infection expérimentale sont non relevants parce que très éloignés des hôtes naturels de l'infection, c'est-à-dire le chien et l'homme, hôtes ayant un impact considérable en terme de santé vétérinaire et humaine. De plus, comme indiqué précédemment, ces travaux ne concernent quasiment qu'une leishmaniose cutanée et notamment *L*. *major* (principal modèle d'infection disponible chez la souris), pour laquelle les études ne sont pas transposables à la leishmaniose viscérale.

La difficulté donc pour atteindre l'objectif de la présente invention est d'une part de conduire une étude sur la leishmaniose viscérale; et d'autre part d'obtenir des résultats transposables avec efficacité et innocuité au chien et à l'homme.

Pour être utilisable chez un hôte naturel, un vaccin contre la leishmaniose doit satisfaire à plusieurs critères généraux :
- posséder une bonne innocuité (absence de toxicité locale et générale du candidat vaccinal, absence de toxicité liée à la réponse immune induite).
- conférer un excellent niveau de protection chez un hôte naturel de l'infection. L'ensemble des travaux de recherche portant sur l'immunologie des leishmanioses s'accorde à dire que la polarisation des réponses immunitaires vers une voie de type Th1 est responsable de l'état de protection.
- avoir un faible coût. Les leishmanioses sont des maladies parasitaires dites « négligées » car elles touchent les populations dés pays les plus pauvres. II est donc important de rendre les nouveaux moyens de prévention accessibles aux populations concernées.
- présenter une bonne stabilité pour une dissémination maximale du vaccin dans les pays pauvres où sévit principalement la leishmaniose
- avoir une bonne faisabilité industrielle. Un vaccin synthétique à tous les avantages industriels face à la production d'un vaccin nécessitant la-culture en masse des parasites.

Deux autres critères d'éligibilité plus particuliers devraient aussi être pris en compte :
- la capacité que possède un vaccin à induire des réponses immunitaires permettant de distinguer en zone d'endémie un hôte vacciné d'un hôte infecté. En effet, comme pour de nombreuses maladies parasitaires, le diagnostic clinique reste aléatoire car les symptômes sont peu spécifiques, souvent absents (il existe de nombreux porteurs asymptomatiques) et n'apparaissent vraiment que lors d'une phase très avancée de l'infection, qui devient alors extrêmement difficile à traiter. Le diagnostic sérologique qui consiste à mettre en évidence des anticorps spécifiques circulants est pratiqué en routine (technique d'immunofluorescence indirecte). Un vaccin lorsqu'il est administré, génère en général des anticorps spécifiques qui très souvent peuvent être aussi produits lors d'une infection. La présence de tels anticorps chez un hôte peut alors être confondante dans le sens où infection et vaccination possèdent la même signature.
- l'avantage de conférer une immunoprotection croisée pour les différentes leishmanioses. Le fait de cibler comme vaccin un ou des antigène(s) commun(s) à toutes les espèces de leishmanies devrait sans aucun doute représenter un réel avantage en terme de vaccination croisée.

La présente invention répond remarquablement et avantageusement à l'ensemble de ces conditions.

Récemment, un candidat vaccinal composé des antigènes d'excrétion sécrétion (AES) de promastigotes de L. infantum formulé avec le Muramyl dipeptide a été proposé (Brevets FR 2825633). Un essai de vaccination chez le chien a permis d'établir un modèle d'étude immunitaire *in vivo* à *Leishmania infantum* chez le chien, hôte réservoir naturel de la leishmaniose viscérale. Celui-ci est en effet naturellement réceptif au cycle de *L. infantum*/*chagasi* et représente une source potentielle de parasites transmissibles à l'homme. Les études d'innocuité chez le chien (phase. I) ont montré une très bonne tolérance locale et générale du candidat vaccinal. Aucune toxicité liée à la réponse immunitaire induite n'a été révélée au cours des différents protocoles d'innocuité. L'évaluation de son efficacité a montré qu'il conférait 100% de protection à l'infection d'épreuve (phase II, Lemesre et al, Vaccine 23, 2005, 2825-2842) et, fait remarquable, protégeait les chiens soumis à l'infection naturelle de deux saisons de transmission en zone d'endémie (étude portant sur 414 chiens recrutés par 18 cliniques vétérinaires) avec un pourcentage d'efficacité proche de 90 % (phase III, Lemesre et al, Vaccine 25, 2007, 4223-4234).

La vaccination par les AES contrôle non seulement le développement de la maladie mais aussi diminue significativement la charge parasitaire chez le chien et ainsi contribue à l'interruption de la transmission des leishmanies.

L'ensemble dés résultats conforte donc solidement le fait que le candidat vaccinal (combinaison des AES de promastigotes de *L. infantum* avec l'adjuvant MDP) protège le chien de la, leishmaniose viscérale à *L. infantum* en induisant durablement une polarisation de la répons à médiation cellulaire vers un profil de type Th1 conduisant à la production d'IFN-g, capable d'activer le macrophage et d'induire la production du monoxyde, d'azote (NO) provoquant la tuerie des amastigotes intracellulaire par apoptose (Brevets FR 2825633, et WO 2004011500).

Parallèlement aux essais immunoprophylactiques, des essais immunothérapeutiques ont donné des résultats prometteurs (brevet WO2004011500. Les chiens vaccinés induisaient une réponse anticorps spécifique et durable d'isotype IgG2, de titre faible et principalement dirigée contre un immunogène majeur de 54 kDa comparativement au groupe placebo. L'immunogène majeur des AES a été identifié par génie génétique comme appartenant à la famille des « promastigote surface antigen » encore appelé PSA.

Ces dernières sont caractérisées par la présence d'un motif répété riche en leucine (Leucine Rich Repeat, LRR) et d'une partie carboxyterminale riche en résidus sérine/thréonine.

On connaît le document Lohman/Mc Mahon Pratt (1990) PNAS 87 : 8393-8397, qui se rapporte au clonage et au séquençage du gêne encodant pour la protéine native GP46/M2 de la forme promastigote de *Leishmania amazonensis,* et relève son implication dans l'immunisation contre les leishmanioses notamment de forme cutanée, chez les modèles expérimentaux murins.

Ce document observe que la protéine native GP46 conserve une conformation dans l'espace tertiaire ou quaternaire, et est immunoprotectrice chez la souris dans un modèle cutané de leishmaniose. Cette observation conduit naturellement le chercheur à penser que cette conformation particulière est impliquée dans le caractère immunodominant de la protéine. Des recherches ont d'ailleurs été menées en ce sens, consécutivement audit document : Sjölander and al. (1998) PH : S0264-410X/98. Cette étude a permis d'observer que la protéine GP46, produite sous forme recombinante dans un système bactérien (*E*. *coli*), système ne tenant pas compte des structures, n'est plus protectrice et perd son efficacité.

Ce qui, en toute logique, conduit à penser que la conformation dans l'espace, secondaire, tertiaire ou quaternaire de la protéine GP46, est importante dans l'efficacité protectrice de ladite protéine.

Or, à l'encontre de ces publications; les inventeurs de la présente demande ont continué leurs travaux sur des peptides de synthèse, qui de par leur taille et leur méthode de préparation ne peuvent présenter que des avantages.

L'intérêt de tels peptides est bien évidemment multiple : évite les problèmes liés à l'isolement de séquences peptidiques, réduit le coût et le temps de mise en oeuvre de cette opération donc augmente la productivité avec un gain financier évident, permet de maîtriser les risques d'infection. De tels peptides répondent de façon remarquable à l'ensemble des conditions citées précédemment : vaccin stable à bas prix et efficace, adapté aux pays pauvres où sévit principalement la leishmaniose.

Bien que la PSA (PSA-2) ait été initialement identifiée chez les formes promastigotes, différentes études semblent indiquer qu'elle est présente aussi chez la forme amastigote. De plus, il a été récemment démontré que cette PSA était même excrétée sécrétée par les deux principaux stades parasitaires et présentait des propriétés immunologiques remarquables dans le sens où des chiens immunisés avec des AES de promastigotes de L.infantum étaient capable d'induire une réponse anticorps spécifique d'isotype IgG2 spécifiquement dirigée contre la PSA excrétée sécrétée (brevet WO2004011200.

Chez *Leishmania* aucun rôle biologique fonctionnel ne leur a été attribué alors que chez d'autres pathogènes comme les bactéries, des protéines à LRR tels que InIB, Yop, et Ssph sont impliquées dans la virulence et la pathogénicité de ces organismes (Marino et al., 2000. PNAS. 97(16):8784-8788).

Fait remarquable, chez les plantes, elles interviennent dans la résistance à différents pathogènes. Très récemment, les études sur l'implication de la PSA (Promastigote Surface Antigen) des leishmanies au niveau de l'adhésion cellulaire (Kedzierski et al., 2004, J Immunol., 172 : 4902-6), de la résistance des parasites à la lyse par le complément (Lincoln et al., Mol Biochem Parasitol. 2004, 137:185-9) et son expression différentielle au cours du cycle de vie des leishmanies (Handman et al., 1995, Inf. Immun., 63 :189-200) ont tendu à montrer que la PSA constitue une composante vitale et essentielle au développement des leishmanies.

La PSA-2 native de *L. amazonensis* confère chez la souris un bon niveau de protection à l'encontre d'une infection expérimentale à *L*. *major* en induisant une immunité protectrice à médiation cellulaire de type Th1 (Handman et al., 1995. Infect. Immun. 63:4261-4267). Par contre, une protéine PSA recombinante produite dans un système d'expression bactérien ne confère pas cette protection, laissant suggérer que les modifications post-traductionnelles de la protéine constitue une étape indispensable à son activité protectrice (MacMahon Pratt, 1996).

Plus récemment, l'utilisation d'un couple de peptides (sous la forme de lipopeptides) de 16 acides aminés chacun confère chez le chien, à l'encontre d'une infection d'épreuve, une immunoprotection similaire à celle développée lors de l'immunisation avec les AES. Les chiens ainsi vaccinés présentent une réponse à médiation cellulaire envers *Leishmania infantum* (brevet FR N° 01/11942).

Par contre, ce vaccin peptidique formé de 2 peptides est incapable de générer une réponse anticorps spécifiques IgG2 importante permettant de distinguer en zone d'endémie un hôte vacciné d'un hôte non vacciné mais infectés. D'autres solutions au probléme de l'immunisation contre la leishmania on été proposées en WO 200605823. et WO 03025012.

La présente invention permet de répondre avantageusement à cet inconvénient majeur notamment grâce à un peptide de synthèse non natif cyclisé par pont disulfure.

Ce peptide peut être utilisé en vaccination seul ou en association avec d'autres peptides.

Toutefois, selon un mode préféré de réalisation de l'invention, c'est utilisé en association avec les deux peptides.de synthèse non natifs décrits dans le brevet FR 2839767 et à un adjuvant induisant de préférence une réponse à médiation cellulaire, que l'on obtient des résultats optimaux en vaccination. Les résultats des tests conduits en vaccination seront exposés ultérieurement.

Ce mélange de trois peptides et d'un adjuvant forme un complexe inventif qui permet de répondre avec une grande efficacité aux inconvénients et insuffisances des modèles proposés antérieurement.

A noter que la forme cyclisée dudit peptide est primordiale dans l'efficacité du complexe selon l'invention. Lés études menées par les inventeurs ont pu en effet établir que la forme non cyclisée de ce peptide n'induit qu'une stimulation Th1 très partielle et présente une efficacité nettement insuffisante, que le peptide soit utilisé seul ou en association avec d'autres peptides. Les inventeurs ont donc conduit des études afin de tenter de suppléer à cette insuffisance et ont ainsi découvert que la forme cyclisée décrite ci-après dudit peptide procurait l'avantage requis.

Selon l'invention, le peptide cyclisé par pont disulfure est composé d'une séquence de 34 acides aminés et désigné : E34P.

Ce peptide de 34 acides aminés (E34P) est composé de la séquence suivante (SEQ ID N°1) :
E-D-E-H-K-G-K-Y-C-R-L-G-N-D-C-R-T-T-E-P-T-T-T-A-T-P-R-G-T-P-T-P-A-P

Ce peptide est cyclisé par pont disulfure entre la cystéine en position 9 et la cystéine en position 15.

Le peptide E34P est avantageusement capable d'induire une réponse anticorps importante permettant de discriminer en zone d'endémie un hôte vacciné d'un hôte non vacciné mais infecté. Cette cyclisation du peptide est essentielle pour l'apparition des anticorps spécifiques IgG2, la cyclisation en pont disulfure entre les cystéines 9 et 15 induisant une conformation spécifique responsable de cette synthèse IgG2. En effet, la non cyclisation du peptide en Cys 9/ Cys 15 n'induit qu'une synthèse peu importante en IgG2 parfois difficile à déceler.

Selon le mode de réalisation préféré de l'invention, le peptide E34P selon l'invention est mélangé à un deuxième et troisième peptides de synthèse non natifs, chacun composé d'une séquence de 16 acides aminés respectivement désignée : A16E et A16G, ou de dérivés de celles-ci, ainsi qu'à un adjuvant.

On entend par « dérivés des séquences A16E ou A16G », l'ensemble des analogues structuraux des acides aminés constitutifs desdites séquences, connus et considérés comme tel par l'homme de l'art.

Le peptide de 16 acides aminés A16E est composé de la séquence suivante (SEQ ID N°2) :
A-A-R-C-A-R-C-R-E-G-Y-S-L-T-D-E
dans laquelle C peut être remplacé par S et L par I.

Le peptide de 16 acides aminés A16G est composé de la séquence suivante (SEQ ID N°3) :
A-A-S-S-T-P-S-P-G-S-G-C-E-V-D-G
dans laquelle C peut être remplacé par S et L par I.

Ces deux derniers peptides sont capables d'induire une stimulation des lymphocytes T de type Th1 assurant la protection contre la leishmaniose viscérale.

Associé à un adjuvant induisant de préférence une réponse à médiation cellulaire, un tel triplet de peptides présente tous les avantages cités précédemment (pas de culture, protection chez un hôte naturelle de l'infection, marqueur de vaccination, immunité croisée, abordable aux populations concernées...). L'ensemble peptides E34P, A16E, A16G, et adjuvant forme le complexe de peptides selon l'invention.

L'adjuvant associé à ces séquences peptidique est de préférence du QA21, de la saponine, de la quil-A, ou tout autre dérivé de ceux-ci connu de l'homme du métier, tel que le QS21.

De manière préférée, les séquences peptidiques E34P, À16E et A16G sont associées dans un rapport E34P/A16E/A16G : 10/25/25.

Le complexe peptidique selon l'invention induit avantageusement une production importante d'anticorps d'isotype IgG2 spécifiques du peptide E34P, ce qui permet de façon remarquable de distinguer les individus vaccinés des individus infectés non vaccinés.

La présente invention est par ailleurs relative à l'utilisation des trois séquences peptidiques E34P, A16E et A16G comme réactifs induisant une stimulation des lymphocytes T de type Th1, notamment pour la prévention et le traitement d'affections liées à un état d'hyperstimulation immédiate et comme réactifs de diagnostic in vitro permettant de détecter les anticorps marqueurs de vaccination, c'est-à-dire les IgG2 spécifiques des fragments peptidiques. La présente invention correspond à un vaccin synthétique de 2^{nde} génération pour le chien (la 1^{ère} génération de vaccin canin correspond au développement de système alternatif antigènes d'excrétion sécrétion AES de promastigotes de *Leishmania infantum,* cité précédemment) adaptable à une formule de vaccination humaine.

Les séquences peptidiques selon l'invention E34P, A16E, et A16G peuvent être rendues immunogéniques par liaisons à des porteurs ou tout autre dérivé (grosses molécules type KLH, lipopeptides type palmitoyl ou dérivés) et sont administrés aux candidats en présence d'un adjuvant, et de préférence le QA21. De façon préférentielle, le rapport peptides/adjuvant est compris entre 3/1 et 3/3.

Dans le cas d'une infection par des leishmanies, les études effectuées chez le chien ont permis de déterminer la dose vaccinale optimale de 20µg de QA21 pour 60µg de peptides injectés. On observe toutefois un début de réponse dès 55µg de peptides injectés.

Le mécanisme d'action du complexe peptidique obtenu selon l'invention est vérifié à l'aide de méthodes spécifiques démontrant que le complexe peptidique innovant agit soit par immunostimulation du système lymphocytaire de type Th1 avec production d'Ig G2, soit par immunomodulation d'un type Th2 vers un type Th1. D'autre part, le rôle neutralisant des IgG2 spécifiques est caractérisé par l'inhibition de la prolifération des formes amastigotes ou promastigotes de Leishmania.

Cette étude de l'état immunitaire de type Th1 est effectuée sur des chiens, après vaccination et également sur des chiens leishmaniens après traitement avec ce complexe peptidique.

### Méthode d'épreuve infectieuse :

L'épreuve infectieuse consiste à injecter en intraveineuse 10⁸ promastigotes en phase métacyclique traités au complément de chien sain et de 5.10⁸_{.} macrophages péritonéaux de chien sain, infectés in vitro par des amastigotes.

Les promastigotes et les macrophages infectés sont dilués dans du sérum physiologique stérile pour un volume final de 1,5 ml. Ce mélange est effectué juste avant l'injection.

Un examen parasitologique est effectué à partir d'échantillon prélevé directement sur le chien.

Un frottis à partir de la ponction de moelle osseuse est effectué sur lame. Ce frottis, une fois fixé au méthanol est coloré au May Grümwald. Giemsa et observé au microscope à immersion (x 1000).

Des prélèvements de moelle osseuse sont mis en culture dans le milieu de culture biphasique NNN (Novy and Mac Neal, 1904, 4. Infec. Dis., 1 : 1-30) dont du RPMI 1640 additionné de 20 % de sérum de veau foetal décomplémenté constitue la phase liquide. Des repiquages à l'aveugle sont réalisés tous les quatre à six jours. Les cultures sont régulièrement observées en microscopie photonique (x 400) pendant 20 mn.

Les parasitémies sont quantifiées comme suit :
+/- : formes allongées-réfringentes immobiles
+ : 1 à 5 formes promastigotes mobiles/champs
++ : > 5 formes promastigotes mobiles/champs
+++ : culture à confluence

### Mise en évidence d'une immunité à médiation cellulaire de type Th1.

Cette mise en évidence s'effectue selon deux techniques :
- Titrage de l'activité leishmanicide des monocytes selon la méthode décrite antérieurement (Brevet FR 2825633) ;
- Détection des Ig G2 de chiens, spécifiques du peptide cyclisé E34P et de la partie carboxyterminale de la PSA.

Cette détection s'effectue par méthode ELISA selon la technique de microtitration de Kweider et al. (J. Immunol, 1987, 138, 299) en utilisant un conjugué anti IgG2. Pour cette méthode, le peptide est biotinylé avant coating sur microplaques. Cette liaison à de grosses molécules de type biotine a notamment pour effet de rendre le peptide cyclisé E34P plus antigénique. Nous utilisons également comme antigène la protéine recombinante codant pour la partie carboxyteminale de la PSA.

Parallèlement à cette étude de l'état Type Th1, un suivi sérologique par immunofluorescence classique utilisant des lames coatées par des promastigotes (méthode de référence sérologique pour la leishmaniose canine) est effectué.

La détection des anticorps d'isotype IgG2 spécifiques du peptide cyclisé E34P et de la partie carboxyterminale de la PSA permet notamment de suivre la réponse immune chez les mammifères vaccinés ou traités, et donc de différencier les mammifères infectés ou malades, des mammifères non infectés ou non malades mais vaccinés ou traités, et de suivre l'efficacité d'une vaccination ou d'un traitement chimiothérapeutique et/ou immunothérapeutique.

Le peptide cyclisé E34P ou le complexe de peptides de synthèse selon l'invention peut être utilisé pour la fabrication d'un médicament, d'un vaccin, ou d'un réactif de diagnostic in vitro et in vivo, pour l'induction de la production d'anticorps spécifiques d'isotype IgG2 spécifique du peptide cyclisé E34P permettant de distinguer un mammifère infecté ou malade, d'un mammifère non infecté ou non malade mais vacciné ou traité, et pour l'induction ou le diagnostic chez les mammifères d'anticorps spécifiques et plus particulièrement d'isotype IgG2, reliés à un état immunitaire de type Th1.

Le peptide cyclisé E34P ou le complexe de peptides de synthèse selon l'invention peut être conditionné sous une forme telle qu'il peut être administré par différentes voies : sous cutanée, intradermique, intramusculaire, intraveineuse, parentérale et orale.

Il peut également être utilisé comme produit de diagnostic in vitro et être inclus dans un kit de diagnostic. Dans une telle hypothèse, le produit de diagnostic in vitro est caractérisé en qu'il contient le peptide cyclisé E34P ou le complexe de peptides selon l'invention, pour la détection d'une réponse humorale et particulièrement des IgG2, permettant notamment la mise en évidence d'un état immunitaire de type Th1 et d'un état d'hyperstimulation retardée, le suivi de la réponse immune chez les mammifères vaccinés ou non vaccinés mais traités, et le suivi de l'efficacité d'une vaccination ou d'un traitement chimiothérapeutique et/ou immunothérapéutique

Le caractère innovant du complexe peptidique selon l'invention ne réside pas seulement dans l'induction d'une réponse cellulaire de type Th1, mais également dans la production de taux d'anticorps de type IgG2. Ces IgG2 sont détectables par diverses méthodes in vitro, par exemple ELISA, DOT BLOT, WESTERN BLOT, IMMUNOCHROMATOGRAPHIE, LATEX, FLUOROPHORES et toute autre méthode in vitro faisant intervenir un système de conjugué ou autres systèmes de visualisation Ag-Ac.

Dans un kit de diagnostic, les peptides E34P, A16E ou A16G peuvent être liés à un support solide, et par exemple à des membranes de nitrocellulose ou autres polymères, des supports de latex et divers matériels de plastic (polymère).

### Mise en évidence du rôle neutralisant des IgG2 spécifiques :

Inhibition de la croissance des promastigotes et des amastigotes en présence des IgG2 (brevet WO 2004011500)

Le peptide de synthèse non natif cyclisé E34P induit des immunoglobulines de classe IgG2 capables de neutraliser la prolifération des amastigotes et promastigotes de *Leishmania sp i*n vitro

### RESULTATS EN IMMUNOTHERAPIE :

Selon les spécialistes comme PINELLI (PINELLI, E et al, Infect Immun, 1994, 62.229-235) les chiens leishmaniens correspondant à l'activation du système lymphocytaire de type Th2 présentent une réponse en anticorps élevée.

Cette production accrue en anticorps correspond à l'hyper protéinémie et induit l'apparition d'immun complexes entraînant une atteinte rénale (augmentation de la créatinine et de l'urée sanguines).

Nous avons donc essayé de moduler vers un état Th1 en administrant à des chiens parfaitement leishmaniens par voie intra dermo des doses du composé peptidique. Le suivi de l'état immunitaire ainsi que l'observation clinique sont effectuées avant et après le traitement.

### Exemple 1 : chien SENIOR

Un chien de race Braque de Weymar âgée de 6 ans, appartenant à Mr G présente de nombreuses lésions cutanées accompagnées d'un état de fatigue général et un aspect maigre, le tout évoquant une leishmaniose canine.

Le vétérinaire, le Dr GH diagnostique une leishmaniose. Ce diagnostic est confirmé par une observation directe au microscope de leishmanies à partir d'un calque cutané et une analyse sérologique qui donne un titre en immunofluorescence leishmaniose positif à 1/1600.

L'analyse de l'état immunitaire avant toute injection permet d'affirmer que le chien est bien dans un état immunitaire de type Th2 avec un titre en anticorps élevé ainsi qu'un test leishmanicide négatif. Nous instaurons une immunothérapie qui consiste à effectuer par voie intradermo 3 injections de 60µg de peptides (25µg A16E, 25µg A16G, 10µg E34P) et 20µg de QA21, chaque injection étant espacée de 3 semaines.

Une semaine après la troisième injection le chien SENIOR retrouve de l'appétit et une certaine vitalité. Le Dr GH commence à observer une légère amélioration cutanée.

Un mois après la dernière injection, SENIOR a retrouvé un aspect clinique normal avec notamment une augmentation de poids de 1 kg et une disparition à 70% de toutes lésions cutanées. L'analyse dé l'état immunitaire permet d'affirmer une baisse de titre en anticorps anti leishmania qui descend à 1/400 en immunofluorescence. Parallèlement, l'effet leishmanicide est positivé. De plus, après traitement, le chien SENIOR présente des IgG2 spécifiques du peptide cyclisé E34P et de la partie carboxyterminale de la PSA, dosées par méthode ELISA. Ces IgG2 spécifiques étaient absentes avant tout traitement.

Une recherche de parasites par culture sur milieu NNN s'avère négative. 8 mois après le traitement le chien SENIOR ne présente aucune modification. Les analyses biologiques permettent d'affirmer que SENIOR est toujours en état immunitaire Th1.

### Exemple 2 : Chien THEO

Un chien mâle de race Epagneul breton, THEO âgé de 5 ans appartenant à Mr K présente les signes cliniques spécifiques de la leishmaniose. Selon le Dr DM, présence de nombreuses squames brillantes, dépilation péri oculaire droite, lésions ulcéreuses au niveau du coude postérieur droit et un état de fatigue prononcé avec notamment un faciès de chien âgé. Les analyses biologiques avec notamment une sérologie leishmaniose positive au 1/800 en immunofluorescence confortent le diagnostic clinique.

Une immunothérapie consistant à effectuer par voie intradermo 3 injections de 60µg de composé vaccinal peptidique (25µg A16E, 25µg A16G, 10µg E34P) et 20µg de QA21, chaque injection étant espacée de 3 semaines, est instaurée. L'analyse de l'état immunitaire avant toute injection démontre que le chien THEO développe un système immunitaire de type Th2 avec une parasitologie fortement positive à partir de la moelle osseuse.

Un mois après la dernière injection, les signes cliniques leishmaniens de THEO rétrocèdent avec notamment une cicatrisation des lésions ulcéreuses, une disparition importante des squames ainsi qu'une dépilation péri oculaire presque inexistante. La sérologie présente une baisse en immunofluorescence au 1/400. Par contre l'analyse de la réponse cellulaire permet d'affirmer que THEO présente un état Th1 actif avec un effet leishmanicide assez important.

Parallèlement, la parasitologie s'est négativée (culture de moelle osseuse en milieu NNN).

Au niveau humoral, le chien THEO présente des IgG2 spécifiques du peptide cyclisé E34P et de la partie carboxyterminale de la PSA après le traitement, IgG2 dosées en ELISA.

La présente invention consiste donc bien en un composé peptidique thérapeutique qui induit le passage d'un état immunitaire de type Th2 avec production importante d'anticorps qui exacerbe les manifestations cliniques vers un état immunitaire de type Th1 entraînant la guérison et accompagné d'IgG2 spécifiques notamment du peptide cyclisé E34P.

### RESULTATS EN VACCINATION DU COMPLEXE PEPTIDIQUE:

Le complexe de peptides est testé sur 14 chiens répartis en 7 groupes, parfaitement sains présentant une sérologie leishmaniose négative, une parasitologie négative, une absence de réponse cellulaire à Leishmania et une absence d'Ig G2 spécifiques du peptide E34P cyclisé. Dans l'établissement des groupes de chiens, il est notamment prévu de comparer le complexe peptidique avec le peptide E34P cyclisé du complexe peptidique avec le complexe E34 NON cyclisé et de comparer le peptide E34P cyclisé seul au peptide E34P non cyclisé seul.

Les 14 chiens ont vécu dans le Nord de la France (Auxerre), zone non endémique, et ont été rapatriés dans le Sud Est de la France, quatre semaines avant la vaccination en période hivernale (période ne présentant pas de vecteurs phlébotomes à leihmania). Parmi les 7 groupes, 2 groupes de chiens recevront le peptide E34P (1 groupe avec E34P cyclisé et 1 groupe avec E34P NON cyclisé), 3 groupes de chiens recevront le mélange peptidique (A16E, A16G et E34P cyclisé) avec notamment 3 concentrations différentes du peptide E34P cyclisé. Un autre groupe de chiens recevra le mélange peptidique (A16E, A16G et E34P NON cyclisé).
- groupe témoin (placebos)
   Chien N° 1 : Témoin négatif : beagle mâle de 3 ans
      Chien N°8 : Témoin négatif : beagle mâle de 3 ans
- groupe A : chiens vaccinés avec les 3 peptides et 1 adjuvant aux concentrations suivantes:
   A16E : 25µg
      A16G : 25µg
      E34P cyclisé : 25µg
      adjuvant QA21 : 20µg
   Chien N° 2 : beagle femelle de 8 ans
   Chien N° 7 : beagle femelle de 7 ans
- groupe B : chiens vaccinés avec les 3 peptide et 1 adjuvant aux concentrations suivantes:
   A16E : 25µg
      A16G : 25µg
      E34P cyclisé : 10µg
      adjuvant-QA21 : 20µg
   Chien N° 3 : beagle femelle de 7 ans
      Chien N° 6 : beagle femelle de 7 ans
- groupe C : chiens vaccinés avec les 3 peptides et 1 adjuvant aux concentrations suivantes:
   A16E : 25µg
      A16G : 25µg
      E34P cyclisé : µg
      adjuvant QA21 : 20µg
   Chien N° 4 : beagle femelle de 9 ans
      Chien N° 5 : beagle femelle de 9 ans
- groupe D : chiens vaccinés avec les 3 peptides et 1 adjuvant aux concentrations suivantes :
   A16E : 25µg
      A16G : 25µg
      E34P non cyclisé en pont disulfure : 10µg
      Adjuvant QA21 : 20µg
   Chien N°9
      Chien N°10
- groupe E : chiens vaccinés avec 1 peptide E34P cyclisé et 1 adjuvant aux concentrations suivantes :
   E34P cyclisé : 10µg
      Adjuvant QA21 : 20µg
   Chien N°11
      Chien N°12
- groupe F : chiens vaccinés avec 1 peptide E34P NON cyclisé et 1 adjuvant
   aux concentrations suivantes :
   E34P non cyclisé : 10µg
      Adjuvant QA21 : 20µg
   Chien N°13
      Chien N°14

Le schéma d'injection vaccinale est le suivant (sem. = semaine) :

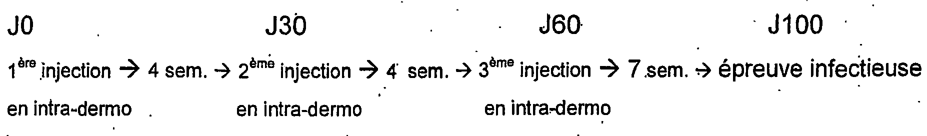

Un suivi clinique des 14 chiens est effectué toutes les 2 semaines.

Les analyses biologiques sont effectuées :
- avant toute injection
- 1 mois après la 3^{ème} injection.

Les analyses biologiques consistent en :
- sérologie IgG2 spécifiques du peptide cyclisé E34P et de la partie carboxyterminale de la PSA ;
- sérologie leishmaniose : immunofluorescence quantitative des IgG totales (signe d'infection) ;
- activité leishmanicide des monocytes (réponse à médiation cellulaire).

Il faut ajouter à ces analyses, la recherche des *Leishmania* par observation directe au microscope et culture sur milieu NNN à partir de moelle osseuse après l'épreuve infectieuse.

Cette recherché des Leishmania s'effectue 4 mois après l'épreuve infectieuse.

Aucune manifestation clinique importante n'est apparue pendant toute cette étude.

Avant toute injection, les 14 chiens présentent des sérologies IgG totales et parasitologies négatives.

Le rôle des IgG2 sur la viabilité et la croissance des promastigotes et des amastigotes de Leishmania est analysé avec les immunosérums de chiens vaccinés avec le mélange comportant le peptide E34P cyclisé et les sérums de chiens placebo.

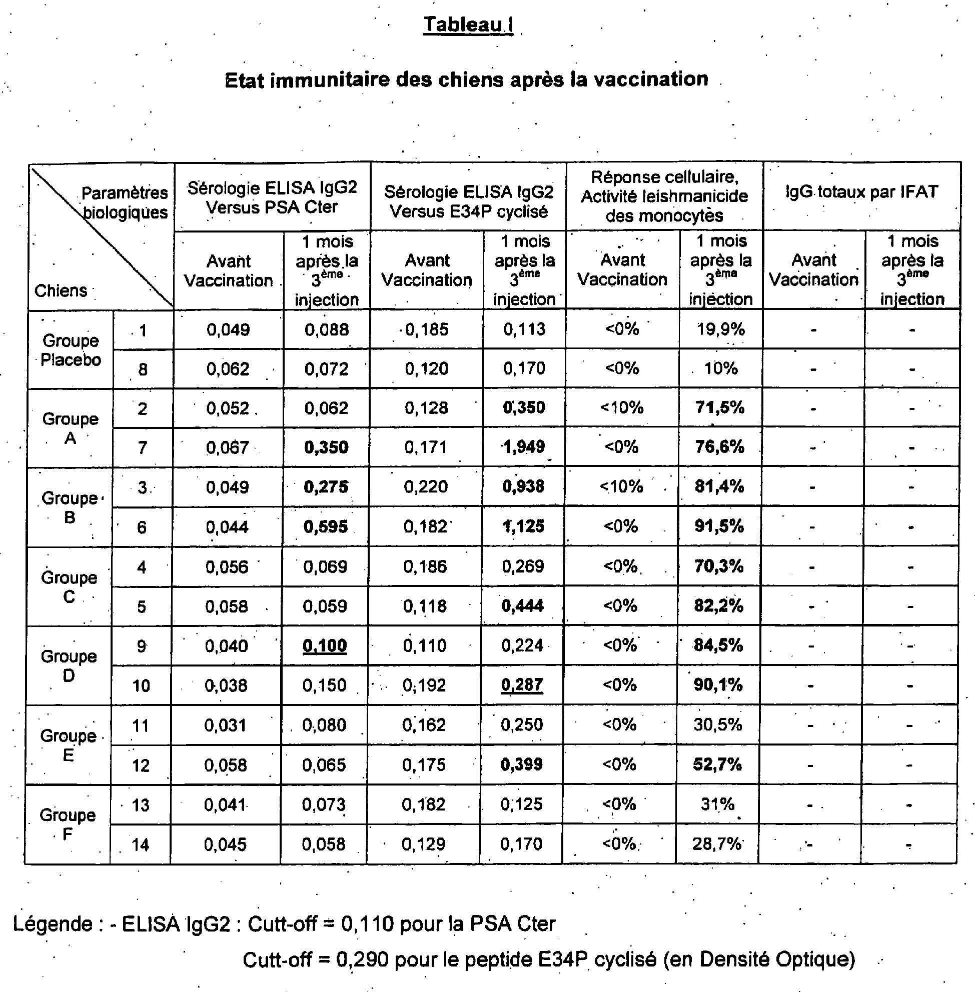

- Activité leishmanicide des monocytes : exprimée en pourcentage d'inhibition de l'index parasitaire (Brevet FR 01/07606) : Cutt-off = 40%
- IgG totaux IFAT Cutt Off ≥ 1/100 (immunofluorescence indirecte)
   Cases grisées = résultats positifs
   Chiffres soulignés = résultats légèrement positifs

Selon le Tableau I, le complexe à 3 peptides avec notamment 10µg du peptide cyclisé E34P (groupe B : chiens 3 et 6) induit bien une immunité cellulaire de type Th1 avec induction d'anticorps d'isotype IgG2 spécifiques. Le peptide cyclisé E34P seul n'induit qu'une réponse partielle. Le peptide E34P NON cyclisé est moins efficace que le E34P cyclisé.

**Tableau II**

| **Sérologie IgG totales et parasitémies effectuées, 4 mois après l'épreuve infectieuse.** | | | | |
|---|---|---|---|---|
| Chiens | | Sérologie IgG Totales (IF quantitatives) | Parasitologie (sur ponctions de moelle) | |
| | | | Examen direct | Culture en milieu NNN |
| Groupe Placebo | 1 | 1/100 | - | ++ |
| | 8 | 1/100 | + | ++ |
| Groupe A | 2 | - | - | - |
| | 7 | - | - | - |
| Groupe B | 3 | - | - | - |
| | 6 | - | - | - |
| Groupe C | 4 | - | - | - |
| | 5 | - | - | - |
| Groupe D | 9 | 1/100 | - | +/- |
| | 10 | - | - | - |
| Groupe E | 11 | 1/200 | - | +/- |
| | 12 | - | - | - |
| Groupe F | 13 | 1/100 | + | + |
| | 14 | 1/100 | - | +/- |

| | | | | |
|---|---|---|---|---|
| Légende : IF = Immunofluorescence (considérée positive si le titre est ≥ 1/100) Parasitologie = culture sur milieu NNN - = absence +/- = rares formes allongées réfringentes immobiles + = 1 à 5 formes promastigotes mobiles/cham ++ = plus de 5 formes promastigotes mobiles/champ +++ = culture à confluence | | | | |

Selon le tableau II, le complexe des 3 peptides avec le peptide cyclisé E34P et l'adjuvant induit bien un état immunitaire de type Th1 protecteur : absence de parasites est d'IgG totales quatre mois après l'épreuve infectieuse, notamment pour les chiens 3 et 6 du groupe B (chiens immunisés avec 10µg E34P, 25µg A16E et 25µG A16G).

D'autre part, le peptide seul E34P cyclisé ne suffit pas pour induire une réponse efficace, d'autre part les résultats du tableau II corrèlent avec ceux du tableau I en ce qui concerne la différence des peptides E34P et E34P **NON** cyclisé : la structure cyclisée du peptide E34P apparaît essentielle.

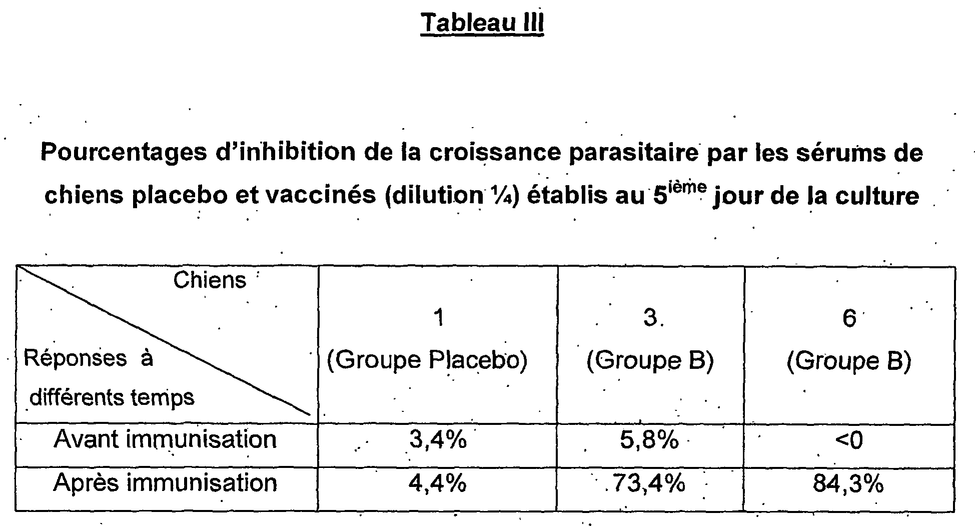

Les résultats obtenus montrent que la réponse IgG2 anti-vaccin des chiens vaccinées avec la formulation 3 peptides avec le peptide cyclisé E34P non seulement accompagne l'immunité protectrice mais aussi exerce une activité anti-parasitaire.

## Revendications

1. Peptide de synthèse non natif cyclisé destiné à induire une production importante d'anticorps spécifiques d'isotype IgG2 permettant de distinguer en cas d'affections liées à un état d'hyperstimulation de type retardé, un animal ou humain infecté ou malade, d'un animal ou humain non infecté ou non malade mais vacciné ou traité, **caractérisé par** la séquence d'acides aminés (E34P) suivante (SEQ ID N°1) :
E-D-E-H-K-G-K-Y-C-R-L-G-N-D-C-R-T-T-E-P-T-T-T-A-T-P-R-G-T-P-T-P-A-P
ladite séquence étant cyclisée par pont disulfure entre la cystéine en position 9 et la cystéine en position 15

2. Complexe de peptides de synthèse non natifs destiné à induire et à caractériser la prévention et/ou le traitement d'affections chez les mammifères dont l'immunité protectrice dépend de la stimulation des lymphocytes de type Th1, et notamment d'un état d'hyperstimulation de type retardé, **caractérisé en ce qu'**il contient :
- la séquence cyclisée (E34P) selon la revendication 1.
- et la séquence (A16E) de 16 acides aminés suivante (SEQ ID N°2) ou des dérivés (analogues structuraux) de celle-ci :
A-A-R-C-A-R-C-R-E-G-Y-S-L-T-D-E
dans laquelle C peut être remplacé par S et L par I.
- et la séquence (A16G) de 16 acides aminés suivante (SEQ ID N°3) ou des dérivés (analogues structuraux) de celle-ci :
A-A-S-S-T-P-S-P-G-S-G-C-E-V-D-G
dans laquelle C peut être remplacé par S et L par I.
- et un adjuvant qui induit de préférence une réponse à médiation cellulaire.

3. Complexe de peptides de synthèse selon la revendication 2, **caractérisé en ce que** l'adjuvant est du QA21, de la saponine, de la quil-A, ou tout autre dérivé de ceux-ci connu de l'homme du métier, tel que le QS21.

4. Complexe de peptides de synthèse selon la revendication 2 ou 3, **caractérisé en ce que** les séquences peptidiques E34P, A16E et A16G sont associées dans un rapport E34P/A16E/A16G : 10/25/25

5. Utilisation du peptide E34P selon la revendication 1 ou du complexe selon l'une quelconque des revendications 2 à 4 pour la fabrication d'un médicament, ou d'un vaccin, ou d'un réactif de diagnostic in vitro et in vivo, pour l'induction ou le diagnostic chez le mammifère d'anticorps spécifiques et plus particulièrement d'isotype IgG2, reliés à un état immunitaire de type Th1.

6. Complexe de peptides de synthèse selon l'une quelconque des revendications 2 à 4, **caractérisée en ce que** les peptides E34P, A16E et A16G sont reliés à des porteurs (grosses molécules type KLH, lipopeptides type palmitoyl ou dérivés) pour les rendre immunogènes.

7. Complexe selon l'une quelconque des revendications 2 à 4 ou 6, conditionné sous une forme telle qu'il peut être administré par différentes voies : sous cutanée, intradermique, intramusculaire, intraveineuse, parentérale et orale.

8. Utilisation du peptide de synthèse non natif cyclisé E34P destiné à induire des immunoglobulines de classe IgG2 selon la revendication 1 pour neutraliser in vitro la prolifération des amastigotes et promastigotes de Leishmania sp.

9. Produit de diagnostic in vitro caractérisé en qu'il contient le peptide cyclisé E34P selon la revendication 1, ou le complexe de peptides selon l'une quelconque des revendications 2 à 4 ou 6, pour la détection d'une réponse humorale et particulièrement des IgG2, permettant notamment la mise en évidence d'un état immunitaire de type Th1 et d'un état d'hyperstimulation retardée, le suivi de la réponse immune chez les mammifères vaccinés ou non vaccinés mais traités, et le suivi de l'efficacité d'une vaccination ou d'un traitement chimiothérapeutique et/ou immunothérapeutique.

10. Kit de diagnostic in vitro comportant le produit de diagnostic selon la revendication 9, **caractérisé en ce que** le peptide cyclisé E34P ou le complexe de peptides est lié à un support solide.

11. Kit de diagnostic in vitro selon la revendication 10 **caractérisé en ce que** le peptide cyclisé E34P ou le complexe de peptides est lié à des membranes de nitrocellulose ou autres polymères, des supports de latex et matériels de plastic (polymère).

## Claims

1. A cyclised non-native synthetic peptide intended to induce substantial production of IgG2 isotype specific antibodies making it possible to distinguish in the case of conditions linked to a state of delayed-type hyperstimulation an infected or ill animal or human from a non-infected and healthy animal or human but vaccinated or treated, **characterised by** the following sequence of amino acids (E34P) (SEQ ID No 1):
E-D-E-H-K-G-Y-C-R-L-G-N-D-C-R-T-T-E-P-T-T-T-A-T-P-R-G-T-P-T-P-A-P
said sequence being cyclised by means of a disulphide bridge between the cysteine at position 9 and the cysteine at position 15.

2. A complex of non-native synthetic peptides intended to induce and characterise the prevention and/or treatment of conditions in mammals, the protective immunity of which depends on the stimulation of the Th1 type lymphocytes, and in particular a state of delayed-type hyperstimulation, **characterised in that** it contains:
- the cyclised sequence (E34P) according to claim 1, and
- the sequence (A16E) of 16 amino acids as follows (SEQ ID No 2) or the derivatives (structural analogues) thereof:
A-A-R-C-A-R-C-R-E-G-Y-S-L-T-D-E
in which C can be replaced by S and L by I,
- and the sequence (A16G) of 16 amino acids as follows (SEQ ID No 3) or the derivatives (structural analogues) thereof:
A-A-S-S-T-P-S-P-G-S-G-C-E-V-D-G
in which C can be replaced by S and L by I,
- and an adjuvant which preferably induces a cell-mediated response.

3. A complex of synthetic peptides according to claim 2 **characterised in that** the adjuvant is QA21, saponin, quil-A, or any other derivative thereof known to the man skilled in the art such as QS21.

4. A complex of synthetic peptides according to claim 2 or claim 3 **characterised in that** the peptide sequences E34P, A16E and A16G are associated in a ratio E34P/A16E/A16G: 10/25/25.

5. Use of the peptide E34P according to claim 1 or the complex according to any one of claims 2 to 4 for the production of a medicament or a vaccine or an in vitro and in vivo diagnostic reactant for induction or diagnosis in a mammal of specific antibodies and more specifically isotype IgG2 linked to an immune state of Th1 type.

6. A complex of synthetic peptides according to any one of claims 2 to 4 **characterised in that** the peptides E34P, A16E and A16G are bound to carriers (large molecules of type KLH, lipopeptides of palmitoyl type or derivatives) to make them immunogenic.

7. A complex according to any one of claims 2 to 4 or 6 packaged in a form such that it can be administered in different ways: subcutaneously, intradermally, intramuscularly, intravenously, parenterally or orally.

8. Use of the cyclised non-native synthetic peptide E34P intended to induce immunoglobulins of class IgG2 according to claim 1 for in vitro neutralisation of the proliferation of Leishmania sp. amastigotes and promastigotes.

9. An in vitro diagnostic product **characterised in that** it contains the cyclised peptides E34P according to claim 1 or the complex of peptides according to any one of claims 2 to 4 or 6 for the detection of a humoral response and particularly IgG2 permitting in particular detection of a Th1 type immune state and a delayed hyperstimulation state, monitoring of the immune response in vaccinated or non-vaccinated but treated mammals, and monitoring the effectiveness of a vaccination or a chemotherapeutic and/or immunotherapeutic treatment.

10. An in vitro diagnostic kit comprising the diagnostic product according to claim 9 **characterised in that** the cyclised peptide E34P or the complex of peptides is bound to a solid support.

11. An in vitro diagnostic kit according to claim 10 **characterised in that** the cyclised peptide E34P or the complex of peptides is bound to membranes of nitrocellulose or other polymers, supports of latex and plastic materials (polymer).

## Patentansprüche

1. Nichtnatives zyklisiertes synthetisches Peptid zur Veranlassung einer erheblichen Produktion von spezifischen Antikörpern des Isotyps IgG2, die es erlauben, im Fall von Erkrankungen, die mit einem retardierten Hyperstimulationszustand verbunden sind, infizierte oder erkrankte Tiere oder Menschen von nicht infizierten oder erkrankten, aber geimpften oder behandelten Tieren oder Menschen zu unterscheiden, **gekennzeichnet durch** folgende Aminosäurensequenz (E34P) (Sequenz-ID-Nr. 1):
E-D-E-H-K-G-K-Y-C-R-L-G-N-D-C-R-T-T-E-P-T-T-T-A-T-P-R-G-T-P-T-P-A-P
wobei die Sequenz **durch** eine Disulfidbrücke zwischen dem Cystein an Position 9 und dem Cystein an Position 15 zyklisiert ist.

2. Komplex von nichtnativen synthetischen Peptiden zur Veranlassung und Charakterisierung der Vorbeugung und/oder der Behandlung von Erkrankungen bei Säugetieren, deren Immunschutz von der Stimulation der Lymphozyten des Typs Th1 und insbesondere von einem retardierten Hyperstimulationszustand abhängt, **dadurch gekennzeichnet, dass** er Folgendes enthält
- die zyklisierte Sequenz (E34P) nach Anspruch 1,
- und die folgende Sequenz (A16E) von 16 Aminosäuren (Sequenz-ID-Nr. 2) oder (analog strukturierte) Derivate davon:
A-A-R-C-A-R-C-R-E-G-Y-S-L-T-D-E
In der C durch S und L durch I ersetzt werden können.
- und die folgende Sequenz (A16G) von 16 Aminosäuren (Sequenz-ID-Nr. 3) oder (analog strukturierte) Derivate davon:
A-A-S-S-T-P-S-P-G-S-G-C-E-V-D-G
in der C durch S und L durch I ersetzt werden können,
- und ein Adjuvans, das vorzugsweise eine zellvermittelte Reaktion bewirkt.

3. Komplex von synthetischen Peptiden nach Anspruch 2, **dadurch gekennzeichnet, dass** das Adjuvans von QA21, Saponin, Quil-A oder jedem anderen dem Fachmann bekannten Derivat stammt, wie z. B. QS21.

4. Komplex von synthetischen Peptiden nach Anspruch 2 oder 3, **dadurch gekennzeichnet, dass** die Peptidsequenzen E34P, A16E und A16G in einem Verhältnis von E34P/A16E/A16G : 10/25/25 miteinander verbunden sind.

5. Verwendung des Peptids E34P nach Anspruch 1 oder des Komplexes nach einem der Ansprüche 2 bis 4 für die Herstellung eines Medikaments oder eines Impfstoffs oder eines In-vitro- und In-vivo-Diagnosereagens für die Induzierung oder die Diagnose von spezifischen Antikörpern und insbesondere des Isotyps IgG2, die mit einem Immunzustand des Typs Th1 verbunden sind, bei Säugetieren.

6. Komplex von synthetischen Peptiden nach einem der Ansprüche 2 bis 4, **dadurch gekennzeichnet, dass** die Peptide E34P, A16E und A16G mit Trägern verbunden sind (großen Molekülen des Typs KLH, Lipopeptiden des Typs Palmitoyl oder Derivaten), um sie zu Immunogenen zu machen.

7. Komplex nach einem der Ansprüche 2 bis 4 oder 6, der in einer solchen Form aufbereitet ist, dass er auf verschiedenen Wegen verabreicht werden kann: subkutan, intrakutan, Intramuskulär, intravenös, parenteral und oral.

8. Verwendung des nichtnativen zyklisierten synthetischen Peptids E34P zum Induzieren von Immunglobulinen der Klasse IgG2 nach Anspruch 1, um in vitro die Vermehrung von Leishmaniose-Amastigoten und -Promastigoten zu neutralisieren.

9. In-vitro-Diagnoseprodukt, **dadurch gekennzeichnet, dass** es das zyklisierte Peptid E34P nach Anspruch 1 oder den Peptidkomplex nach einem der Ansprüche 2 bis 4 oder 6 enthält, für die Erkennung einer humoralen und insbesondere einer IgG2-Antwort, wodurch vor allem der Nachweis eines Immunzustands des Typs Th1 und eines retardierten Hyperstimulationszustands, der Folgeerscheinung der Immunantwort bei geimpften oder nicht geimpften, aber behandelten Säugetieren, und der Folgeerscheinung der Wirksamkeit einer Impfung oder einer chemotherapeutischen und/oder immuntherapeutischen Behandlung ermöglicht wird.

10. In-vitro-Diagnosekit mit dem Diagnoseprodukt nach Anspruch 9, **dadurch gekennzeichnet, dass** das zyklisierte Peptid E34P oder der Peptidkomplex an eine feste Trägersubstanz gebunden sind.

11. In-vitro-Diagnosekit mit dem Diagnoseprodukt nach Anspruch 10, **dadurch gekennzeichnet, dass** das zyklisierte Peptid E34P oder der Peptidkomplex an Nitrocellulose-oder andere Polymer-Membranen, Latex- und Kunststoffmaterial- (Polymer-) Trägersubstanzen gebunden ist.
